# EUROPEAN PATENT APPLICATION

(11) **EP 0 896 999 A1**
(43) Date of publication of application: **17.02.1999**
(21) Application number: 97907282.4
(22) Date of filing: 12.03.1997
(51) Int. Cl.: C12N 5/00

(54) **HUMAN T CELL CLONE SPECIFIC FOR RHEUMATOID ARTHRITIS**

(30) Priority: 13.03.1996 JP 56022/96; 23.10.1996 PC /JP96/03082 T
(71) Applicant: SHIONOGI & CO., LTD., Osaka-shi, Osaka 541-0045 (JP)
(72) Inventor: TOYOSAKI-MAEDA, Tomoko, Kobe-shi, Hyogo (JP); SUZUKI, Ryuji, Ikoma-gun, Nara 636 (JP); TSURUTA, Yuji, Higashiosaka-shi, Osaka 577 (JP); TAKEMOTO, Hiroshi, Kobe-shi, Hyogo 654-01 (JP)
(74) Representative: Baverstock, Michael George Douglas
(86) International application number: JP9700774
(87) International publication number: WO9733977

(57) **Abstract**

A human T cell clone which recognizes the antigen expressed in the synovial cells of patients with rheumatoid arthritis (RA) as HLA-DR-restrictive and is useful for the elucidation of the mechanism of RA onset and the development of methods of treating and preventing RA.

## Description

### FIELD OF THE INVENTION

The present invention relates to an agent useful in diagnosing, preventing and/or treating an autoimmune disease, and particularly, to a T cell clone which is specifically reactive to an antigen presented on a certain HLA-DR which is expressed by a synovial cell of a patient suffering from rheumatoid arthritis (RA).

### PRIOR ART

An immune response, which is primarily a reaction to destroy and eliminate exogenous material invading from external ambient, is usually induced specifically for a non-self molecule (antigen). However, when the recognizability between a self and a non-self molecule is disordered by some reasons (failure in self tolerance), various autoimmune diseases develop, such as systemic erythematodes, pachydermia, multiple sclerosis (MS), rheumatoid arthritis (termed as RA, hereinafter), and the like. RA frequently attacks women after middle-age. Predominant symptoms of RA include the swelling and deformation, pain, and motor function failure in joints, and its histopathological finding is an abnormal outgrowth of synovial tissues lining capsula articularis (joint capsule). At the outgrowing tissues, a large number of immunocompetent cells (neutrophile, macrophage, lymphocyte), as well as a folicle formation of B cells, which are responsible for antibody production, and a population of helper T cells, are observed. Previous epidemiologic survey has shown that RA is associated with HLA-DRB1 allele as a disease-sensitive gene, and that RA frequently attacks women at pre- and post-menopausal period. Pathologically, the infiltration of memory type CD4-positive, CD45RO-positive T cells into lesions (synovial tissue) has been found, suggesting that the helper T cells having a memory for a certain antigen would be highly responsible for the onset of RA.

Nowadays, although non-steroidal anti-inflammatory agents, immunomodulators, and steroid agents are predominately utilized in the therapy for RA, these medicaments may produce various side effects (gastrointestinal injury, nephropathy, and the like). Additional disadvantages in the usage of immunomodulators include the delayed exertion of effect, and besides a possible ineffectiveness due to its repetitive administration, and therefore, the usage of immunomodulators is questionable. Recently, it is begining that immunosuppressants are used in order to control more efficiently the inflammation. Unfortunately, imuunosuppressants may produce severe side effects such as myelopathy, and therefore, there is a large demand of the exploration for a treatment targeting the lesion.

Methods for treating specifically autoimmune diseases include those as recommended in treatment of MS, such as, for example, exhaustion of T cells from body which are reactive to a certain pathogenic autoantigen (for example, vaccination of T cell), re-induction of autoimmune tolerance against the antigen, and the like. Treatment of RA by such methods requires establishment of a RA-associated autoantigen and a T cell clone specific for said antigen so as to prepare a clone of the RA-associated T cells. Isolation of such clone should help to identify a T cell receptor associated with the pathogenesis of RA (also referred to as TCR hereinafter) and a gene encoding the receptor, and to isolate and identify an antigen to be specifically recognized by TCR, thereby to explore the mechanism of the activation of the T cell and the pathogenesis of RA. The final goal of isolation of such clone is believed to contribute to exploitation of treatment, prevention and diagnosis of RA.

Unfortunately, partly because an appropriate animal model for RA has not been available, no report has yet described establishment of the T cell clones which definitely recognize the RA-associated autoantigen, and the antigen associated with the pathogenesis of RA has not been certainly identified, and further, contribution of infiltrating CD4⁺ memory T cells and synovial cells toward the pathogenesis of RA has been still unknown. In general, establishment of T cell clones reactive to a particular antigen can be performed by, for example, the intermittent stimulation with said antigen in the presence of interleukin-2 to prepare T cell lines specifically reactive to the antigen, and by the limiting dilution of the resultant T cell lines. It is, however, very difficult to maintain these clones for extended periods of time without definition of antigen specificity (without stimulation with the defined antigen). Under such circumstances, establishment of RA-specific T cell clones according to the above method is quite impossible, similarly to certain other autoimmune diseases because autoantigen to be associated with the disease has not yet been identified.

### DISCLOSURE OF THE INVENTION

The present inventors have studied preparation of proliferative T cell clones which are associated with the pathogenesis of RA, and successfully established and characterized the T cell clones which specifically recognize the antigen from the synovial cells of RA patients

In particular, the present invention provides the T cell clones which recognize the antigen presented by the synovial cells from patients with rheumatoid arthritis in HLA-DR-restricted manner.

The T cell clones of the present invention proliferate specifically in recognition of the RA-associated antigen on condition that said antigen is presented on the particular genotype of HLA-DR.

The T cell clones of the invention include HLA-DR4- or HLA-DR9-restricted clones, which recognize the RA-associated antigen presented on HLA-DR4 or HLA-DR9.

Examination of the TCR region of the T cell clones of the present invention revealed that the CDR3 regions of TCR Vα chains contain arginine, and their TCR β chains are Vβ 6 family, and that the TCR β chains of some clones are Vβ 12.

In an embodiment of the present invention, the T cell clones are CD4-positive and CD45RO-positive, and proliferate in the presence of the synovial cells from the RA patients bearing HLA-DR4 or HLA-DR9.

As used herein with reference to T cell clone, the term "reactive to" means that a T cell clone proliferate in vivo and in vitro in response to a specific antigen under a defined condition.

As used herein with reference to a particular T cell clone, the term "autologous RA synovial cell" or "self RA synovial cell" refers to a synovial cell derived from the same source (synovial tissue of RA patient) as said particular T cell clone. In contrast, the term "nonself RA synovial cell" refers to a RA synovial cell derived from a source which is different from that of the particular T cell clone. In other words, discrimination between "self" and "nonself" synovial cells is dependent on only identity of the source from which they are derived, and therefore, "nonself RA synovial cell" includes not only a genotype-mismatched synovial cell, but also a HLA-DR-matched synovial cell.

As used herein with reference to T cell clone, the term "autoreactive" means that a T cell clone proliferates in the presence of the autologous RA synovial cell. It should be noted that the meaning of the term "autoreactive" can expand to include the phenomena of proliferation of the T cell clone in the presence of a nonself synovial cells bearing the same genotype of HLA-DR as the T cell clones (a HLA-DR-matched nonself RA synovial cell), in the light of the fact that the "autoreactive" T cell clones of the present invention respond to not only the self RA synovial cells, but also to the nonself synovial cells bearing the same genotype of HLA-DR as the T cell clones, as described herein.

The term "HLA-DR-restricted" means that a T cell clone proliferates in recognition of the RA-associated antigen regardless of whether the antigen is from the self or nonself synovia on condition that said antigen is presented on a certain HLA-DR.

Accordingly, the HLA-DR-restricted T cell clones of the present invention proliferate only when a possible RA-associated antigen is presented on a certain HLA-DR, and they cannot proliferate when the antigen is presented on an allogeneic HLA-DR.

The term "RA-associated antigen" refers to an autoantigen to be considered to be an etiologic agent of rheumatoid arthritis, and typically include a peptide fragment of the antigen which is associated with a certain HLA molecule to be recognized by the T cell, or an intact antigen comprising said fragments.

### BRIEF DESCRIPTION OF THE DRAWINGS

Figure 1 is the flowchart which shows steps for preparing the autoreactive T cell clones.
Figure 2 is the graph which shows inhibitions of the reactivity of the HLA-DR-restricted T cell clones to the autologous synovial cells with the anti-HLA-DR monoclonal antibody (mAb) or anti-TCR αβ mAb.
Figure 3 is the graph which shows the reactivity of the HLA-DR-restricted T cell clones to solubilized antigens from RA synovial cells, and to solubilized antigens from synovial cells of healthy donors and non-RA patients. N-1 and N-3 are from healthy donors, and OA-1 is from arthrosis deformans patients.

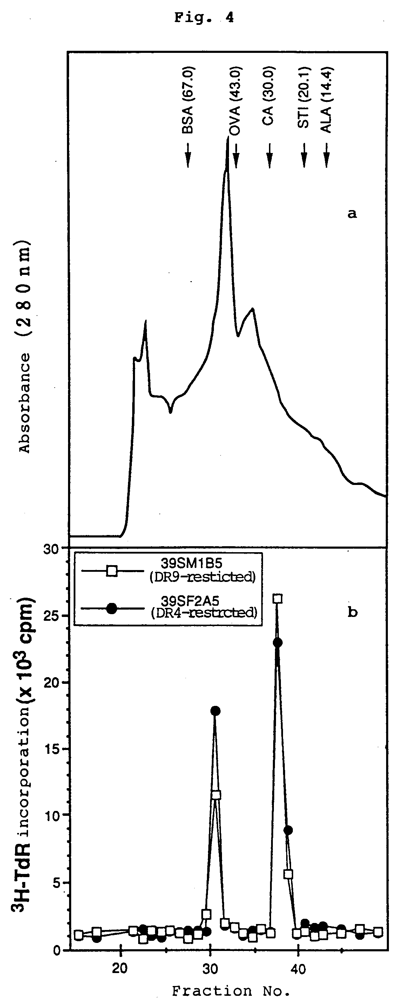

Figure 4a is the chromatograph of the solubilized antigens from RA synovial cells, which includes the elution points of various standards of BSA (bovine serum albumin), OVA (ovalbumin), CA (carbonic anhydrase), STI (soybean trypsin inhibitor), and ALA (bovine milk α-lactoglobulin). HLA-DR-restricted T cell clones (39SM1B5, 39SF2A5). The numbers in the brackets represent the molecular weights of the standards. Figure 4b is the graph which shows the reactivity of the HLA-DR-restricted T cell clones to the chromatography fractions (Fig. 4a). The molecular weights of the antigen fractions (HPLC fractions 31 and 38), which are found to contain the reactivity are estimated to be 50 kDa and 25 kDa, respectively by calibration curve determined from the elution points of the standards.

### BEST MODE FOR CARRYING OUT THE INVENTION

The general description of the procedures for establishing the T cell clones of the present invention and their characters are provided below. The invention is not limited to a T cell clone restricted with a particular HLA-DR, but encompasses RA-specific T cell clones restricted with various HLA-DR, which are obtainable according to the methods described herein.

Preparation of the human T cell clones specific for the antigen expressed by the RA synovial cells, principally, comprises each of the following steps:
(1) culturing and establishing T cell clones derived from synovial tissues or synovial fluids of a patient with rheumatoid arthritis in an appropriate medium;
(2) selecting the T cell clones reactive to the autologous synovial cells from the same patient;
(3) selecting the T cell clones which are not reactive to synovial cells derived from another RA patient which bears the different HLA-DR from the autologous synovial cells; and/or
(4) selecting the T cell clones which are not reactive to cells expressing the same HLA-DR as the autologous synoviocytes, which cells do not express the RA-associated antigen derived from the synovial cells of the patient in the above item (1).

First, synovial tissues and synovial fluids are obtained from RA patients, and are digested with a digesting solution containing collagenase, hyaluronidase, and DNase, to obtain the primary culture of mononuclear cells. Then, T cell clones are selected by limiting dilution, and cultured in an appropriate medium. In the establishment of T cell clones, the well-known media with modifications according as particular intentions can be used, and preferred medium is one developed by the present inventors containing human interleukin-2, culture supernatant of human peripheral blood mononuclear cells stimulated with PHA, and Fetal calf serum. Especially preferred is the medium which comprises 400 ml of AIM-V medium (GIBCO BRL, #87-0112) supplemented with 50-100 ml of RPMI 1640 medium (GIBCO BRL, #22400), 30-60 ml of human T-STIM (10BRMP/ml; BRMP, Biological Response Modifier Program Jurkat IL-2 reference reagent), 250-750 U/ml recombinant human IL-2, 50-100 ml of Fetal calf serum (heat-inactivated by treating 56°C for 30 minutes), and antibiotics (for example, about 100 U of penicillin and about 100 µg/ml streptomycin). This is termed as MIX-H medium, Human T-STIM refers to a culture supernatant of human peripheral blood mononuclear cells stimulated with phytohemagglutinin (Human T-STIM with PHA, Becton Dickinson, # 40045).

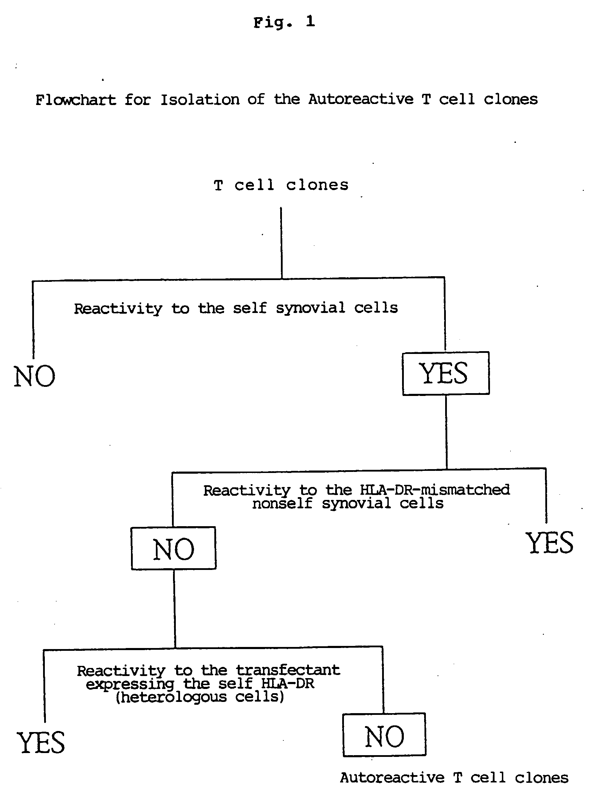

From the T cell clones of each of RA patients established as shown above, the CD4-positive and CD45RO-positive clones are selected. Starting from the clones, the autoreactive T cell clones are prepared according to the method of Figure 1. In the working examples hereinafter, totally eight autoreactive T cell clones are obtained from two patients (termed as RA39 and RA 40). One of the synovial cells from the RA patients, the cell line RA39-sy (HLA-DRB1* 0405/0901), which is starting material, was deposited at the National Institute of Bioscience and Human Technology, Ministry of International Trade and Industry (1-1-3 Higashi, Tsukuba-shi, Ibaraki) on October 3, 1995, under Budapest Treaty on the International Recognition of the Deposit of Microorganisms for the Purposes of Patent Procedure, and given Accession No. FERM BP-5681.

The T cell clones of the invention may be also prepared starting from T cells derived from any tissues other than synovial tissue or synovial fluid. In brief, to T cells considered to proliferate only in response to the antigen (or non-autoproliferative T cell) are added the cells which may be an antigen-presenting cell and solubilized material of the synoviocytes, and the mixture is cultured on an appropriate medium (for example, RPMI 1640 medium supplemented with 10% Fatal calf serum) at 37°C for 10-20 days to provide the T cell lines. Then, the above steps used in separation of the autoreactive clones from the synovial tissues are performed on the resultant T cell lines to provide the desired autoreactive T cell clone.

"Cells which may be an antigen-presenting cell" include, for example, a macrophage, a B cell derived from human, and the like, whereas "T cells considered to proliferate in antigen-specific manner (or non-autoproliferative T cell)" include a T cell, a tissue-infiltrating T cell derived from human peripheral blood, and the like.

Reactivity of T cell clone (antigen-specific proliferating activity) is generally determined on the basis of ³H-thymidine incorporation (CPM), but can be determined by the following methods:
1. Determination of the activity of enzymes involving nucleic acid synthesis, such as DNA polymerase;
2. Determination of the formation of the complex comprising ethidium bromide and DNA;
3. Determination of the glucose consumption in a culture; and
4. Observation of the morphology (counting of viable cells).

As shown in Figure 1, the established T cell clones are first cultured in the presence of the autoreactive RA synovial cells to select the reactive T cell clones, following which the T cell clones which are not reactive to HLA-DR-mismatched synoviocytes are selected. Here, the synoviocytes may be stimulated with γ-interferon to express strongly their HLA-DR.

Then, the clones which are not reactive to the murine fibroblast into which the HLA-DRB1 is introduced are selected (HLA-DR transfectant). This procedure results in the autoreactive clones which recognize the antigen-presented HLA-DR, whereas removal of the clones reactive to the HLA-DR on which the antigen is not expressed.

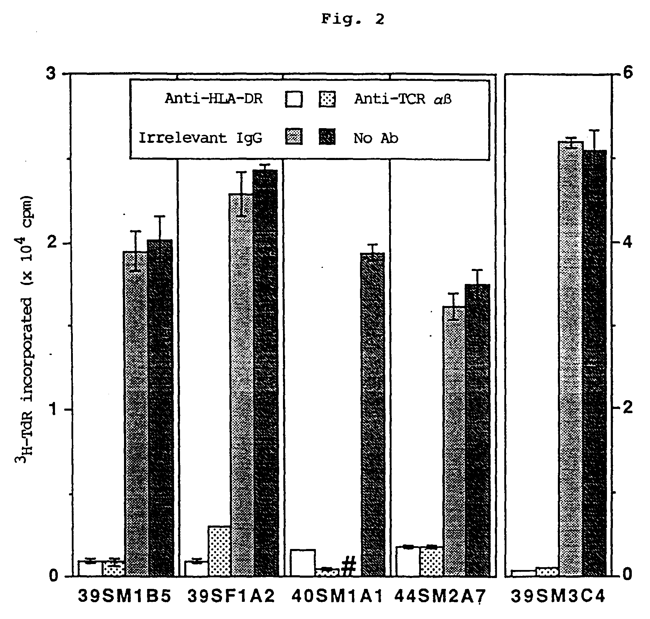

Characteristics of the clones of the present invention are provided blow:
1) The clones proliferate in response to not only self- but also nonself-RA synoviocytes in HLA-DR-restricted manner, and they are not reactive to peripheral blood antigen-presenting cells (PB-APC) from healthy donors which have the same HLA-DR as the self (See Table 1 hereinafter);
2) The clones are not reactive to the self PB-APC;
   The results of the above 1) and 2) show that the T cell clones of the present invention recognize the antigen in HLA-DR-restricted manner, and that the antigen is present exclusively in the synovial cells.
3) The clones are reactive to synovial cells derived from many RA patients (See Table 1);
4) Autoreactivity of the clones is absolutely inhibited by anti-HLA-DR and anti-TCR αβ antibodies (all are murine IgG), whereas it is not affected by irrelevant murine IgG (See Figure 2);
5) Examination of TCR of the clones for its amino acid sequences revealed that some clones have arginine in their CDR3 regions of the TCR α chains, whereas all of them use TCR Vβ 6, and that the other clones have no significant characters in the α chains, whereas all of them use TCR Vβ 12 (See Table 2);
6) In the presence of the antigen-presenting cells from the peripheral blood, the clones are reactive to the solubilized antigen extracted from RA synoviocytes, whereas they are not reactive to the solubilized antigen extracted from non-RA synoviocytes (See Table 3);

The results of the above 3) to 6) show that the T cell clones of the present invention each recognize the common or similar antigen(s), and that the antigen(s) are unique to RA, suggesting the possibility of the presence of universal RA-associated antigen(s), which would commonly exist in many RA patients.

As described above, the autoreactive T cell clones of the present invention comprises established human T cell clones which recognize RA-associated antigen in HLA-DR-restricted manner, and are very useful in exploring the etiology of RA, and in developing treatment, accurate diagnosis, and prevention of RA. For example, the T cell clones of the invention make it possible to detect any RA-associated antigen on the basis of their reactivity to synoviocytes from synovial tissue of patients suspected to suffer from RA. Thus, the present invention also provides a method for detecting RA-associated antigen(s) which comprises using the T cell clones' reactivity as an indicator, and such RA-associated antigen(s) which are detectable according to the method. The method for detecting the RA-associated antigen comprises, for example, obtaining synovial cells from patients suspected to suffer from RA; solubilizing the cells; reacting the solubilized material with the human T cell clones and the cells derived from the patient which may be an antigen-presenting cell; and determining the reactivity of said clones.

Examples of the RA-associated antigens which were detected according to the method of the invention include those estimated as 25 kDa or 50 kDa of molecular weight determined on HPLC over TSK gel G3000SW column eluting with PBS at 0.5 ml/minute. Such antigen(s) obtained as shown above are useful in developing therapeutics of RA specific for the lesion, as well as exploring the etiology, treatment, diagnostics of RA, and the like.

Further, the partial sequence of the TCR of the T cell clones specific for the RA-associated antigen which sequence the present invention has been demonstrated may lead preparation of a molecule having the TCR activity in the amount necessary to perform an examination, by means of preparing the intact TCR molecule or partial peptides which are responsible for the RA-associated antigen recognition, and preparing DNA encoding them or DNA fragments thereof, and introducing the same into a appropriate host cell, and expressing the molecule in compliance with DNA recombinant technology. Such recombinant TCR molecule is useful in exploration of the structure of RA-associated antigen-HLA-DR complex, and besides, further exploration of the RA-associated antigen itself.

Clinically, the RA-associated antigen makes it possible to re-induce self tolerance in the patients, as well as to suppress or extinguish the symptoms. Further, it is understood that T cell clones involving inflammatory could be selectively removed by administering autologous T cell clones or TCR to patients together with an appropriate adjuvant, as shown above with respect to MS. Also, the onset of symptoms could be suppressed by blocking the recognition by T cells of the antigen presented by the synoviocytes with an agent competing with TCR to bind the antigen-HLA-DR complex. Alternatively, it could be possible to treat RA if monoclonal antibody toward the TCR of the T cell clones is prepared, and such monoclonal antibody is administered to the RA patients in order to remove the RA-inducing T cells from the body.

Further, detection of the T cells reactive to the RA-associated antigen allows to diagnose RA. Also, examination of the presence of the antibody toward the RA-associated antigen helps to diagnose RA.

On the other hand, the synovial cells as described herein are useful in not only studying the RA-associated antigen recognized by the HLA-DR4/9-restricted autoreactive T cell clones, but also in screening for the RA involved by the same HLA-DR-restricted T cells.

As shown above, the present invention provide the T cell clones specific for the RA-associated antigens derived from many patients for the first time, and such clones can contribute to the exploration of etiology of RA, and to the development of treatment, prevention and diagnosis of RA.

The following examples are presented for the purpose of further illustration of the invention, and such examples are not intended to be limiting the invention in any respect.

### Example 1 Establishment of T cell clones from diseased synovia of RA patients

### (1) Materials

### 1) Preparation of synovial tissues and fluids

Under sterilized condition, synovial tissues and fluids were obtained from the diseased synovium of the patient (RA39) classified as class 2-4, stage 2-3 according to the 1987 ACR criteria for diagnosis of rheumatoid arthritis (RA) (F. C. Arnett et al., Arthritis Rheum., 31;315 (1988)). The synovial tissues were stored in tubes containing the saline or a medium at 4°C. The synovial fluids were stored in tubes at 4°C. The following procedures were performed under sterilized condition, unless otherwise indicated.

### 2) Preparation of digesting solution

0.5 Mg of collagenase (type IV, Sigma Chemical Co.), 0.5 mg of hyaluronidase (Sigma), and 5 µg of DNase (type I, Sigma) were dissolved in 500 ml of DMEM (Dulbecco's Modified Eagle Medium) (GIBCO BRL, #12430-021 or an equivalent) supplemented with high concentration of glucose (4500 mg/L) at an environmental temperature to provide a digesting solution. Ten ml aliquots of the solution were introduced into polypropylene screw-capped tubes (Corning Costar Co. #430766 or an equivalent), and the tubes were stored in the freezer at -15°C until ready to use.

### 3) Preparation of MIX-H medium

Into 400 ml of AIM-V medium (GIBCO BRL, #87-0112) were supplemented 60 ml of RPMI 1640 medium (GIBCO BRL, #22400), 40 ml of human T-STIM (10BRMP/ml; BRMP, Biological Response Modifier Program Jurkat IL-2 reference reagent), 500U/ml recombinant human IL-2 (Shionogi Co. & Lit.), 50 ml of Fetal calf serum (heat-inactivated by treating 56°C for 30 minutes), and antibiotics (100 U of penicillin and 100 µg/ml streptomycin; GIBCO BRL#15140-015 or an equivalent) so as to prepare MIX-H medium. Human T-STIM refers to a culture supernatant of human peripheral blood mononuclear cells stimulated with phytohemagglutinin (Human T-STIM with PHA, Becton Dickinson, # 40045).

### (2) Digestion of Synovial Tissues

The digesting solution was thawed at 37°C in a heating bath. The saline was removed from the tubes in which the synovial tissues were stored, and 5 g sample of the tissue was teased apart with scissors on a plastic dish. To the new screw-capped tube containing the teased tissue was added cold, serum-free RPMI 1640 medium (5-10 fold volume of the tissue), and the tube was centrifuged at 1000 rpm for 5 minutes at 4°C. Discarding the supernatant from the tube, the thawed digesting solution (10-20 ml) was added to the tube, and then the tube with the specimen was put in a heating bath at 37°C, and incubated for 2-3 hours while shaking gently.

Then, RPMI 1640 medium (2-3 fold volume) was added to the tube, and the tube was allowed to left and to precipitate the undigested tissue, following which the suspension with the synoviocytes was collected. These procedures were repeated two more times, and the collected suspensions were combined, and the combination was centrifuged at 1000 rpm for 5 minutes at 4°C.

The number of the digested cell was counted and the viability of the single cells was checked by trypan blue exclusion test.

### (3) Primary Culture of Mononuclear Cells and Isolation of Synovial cells

An aliquot of the resultant cells was suspended in MIX-H medium, and the suspension was incubated at 37°C in 5% CO₂ atmosphere. The primary culture contained both synoviocytes and tissue-infiltrating mononuclear cells. After two-three weeks of the culture and passage on this condition, the mononuclear cells will be activated and expanded, whereas the few synovial cells will be observed in the culture.

On the other hand, the synoviocytes were expanded by culture in DMEM supplemented with 10% Fetal calf serum and antibiotics. In brief, a portion of the resultant cells as shown in the above (2) was cultured in DMEM containing high concentration of glucose (4500 mg/L) supplemented with 10% Fetal calf serum and antibiotics at the same concentrations as in MIX-H medium at 37°C in 5% CO₂ atmosphere, and the synoviocytes were collected. Synovial cell line RA39-Sy (HLA-DRB1*0405/0901), which expresses both HLA-DR4 and HLA-DR9 from RA39, was deposited at the National Institute of Bioscience and Human Technology, Ministry of International Trade and Industry on October 3, 1995, under Accession No. FERM BP-5681.

### (4) Primary Culture of Mononuclear Cells from Synovial Fluid

About 10 ml of the synovial fluid was transferred into the new screw-capped tube with transfer pipets, and the tube was centrifuged at 2500-3000 rpm for 10 minutes at 4°C until the pellet was observed at the bottom of the tube. The cell-free synovial fluid was removed, and if necessary, the fluid was stored at -15°C. The resultant cells were suspended in MIX-H medium, and the mixture was incubated at 37°C in 5% CO₂ atmosphere. Two-three weeks of the culture and passage on this condition causes the mononuclear cells to be activated and expanded.

### (5) Cloning of T cells from Mononuclear Cell lines

Each of the mononuclear cell lines from the synovial tissues and fluids expanded as described in the above (3) and (4) was observed for the large cluster of the activated cells. When there are few cluster or large proportion of dead cells (>50%), it is necessary to exchange the half of the culture fluid (MIX-H) every 3-5 days while observing repeatedly the cluster.

Allogeneic peripheral blood mononuclear cells (PBMC) were prepared from the heparinized peripheral blood, and incubated with 25 µg/ml mitomycin C in RPMI 1640 (serum free) at 37°C for an hour. These PBMCs were used as the feeder cells. The feeder cells were adjusted to 3 x 10⁵/ml in MIX-H medium, and were dispensed at 5 x 10⁴ cells/100 µl/well on the 95-well round bottom plate.

The cell lines expanded as shown above were washed once with serum-free RPMI 1640 medium, and the cell number was countered with trypan blue staining. The cell volume was adjusted to 1 x 10³/10 ml, 5 x 10³/10 ml, and 1 x 10⁴/10 ml in MIX-H medium, and the cells were dispensed at 10, 50 or 100 cells/100 µl/well on the plate together with the feeder cells.

Then, the plate was incubated at 37°C in 5% CO₂ atmosphere for 2-4 weeks. A week after starting the cloning, the plate was checked. Then, the positive wells in which the cells were increasing could be distinguished, and the number and occurrence time of the positive wells varied from cell line to cell line. The half of the medium was exchanged with a fresh medium every one week.

The expanded cells were transferred into 48-well culture plates, further expanded, and 59 and 53 of the T cell clones were established from the autologous RA synovial tissues and fluids, respectively. Among them, 108 clones were CD4-positive and CD45RO-positive, whereas the remaining 4 clones were CD8-positive.

### (6) Selection for Autoreactive T cell Clones

According to the procedures described in Figure 1, the clones specific for the RA synoviocytes isolated in the above (3) were selected from the CD4-positive and CD45RO-positive T cell clones prepared in the above (5).

The reactivity of the T cell clones to the self or nonself RA synoviocytes was estimated on the basis of ³H-thymidine incorporation (CPM) after culturing them in RPMI 1640 medium supplemented with 10% Fetal calf serum and antibiotics at 37°C for 72-96 hours.
1) Examination of the reactivity to autologous RA synoviocyte (HLA-DRB1*0405/0901) according to the above method provided 17 positive clones.
2) Examination of the reactivity to the allogeneic and nonself RA synoviocyte (HLA-DRB1*0802/0802) provided eight negative clones.
3) The T cell clone which was not reactive to the mouse transfectant expressing the autologous HLA-DR (HLA-DRB1*0405/0901) were obtained. This removed the T cell clone reactive to HLA-DR on which any antigen was not presented, and provided six HLA-DR-restricted clones, which are specifically reactive only to the particular HLA-DR on which the RA antigen was presented.

According to a similar procedure, synovial tissues and fluids were obtained from another patient RA40, and each one of the autoreactive T cell clone from them was prepared. The HLA-DR allele of the patient RA40 was DRB1*0901/1502.

As shown above, eight autoreactive T cell clones were totally prepared.

In order to examine the reactivity of the autoreactive T cell clones of the present invention, the structure thereof, and the characteristics of the RA synoviocytes and RA antigen recognition, the following series of experiments were performed. It should be noted that the results representing the reactivity (proliferating activity) of T cell clones were determined by ³H-thymidine incorporation (CPM) similarly to in Example 1, unless otherwise specified.

### Experiment 1

### (1) HLA-DR restriction of autoreactive T cell clones

Eight autoreactive T cell clones (39SM1B5, 39SM1D4, 39SM3C4, 39SF1A2, 39SF1D1, 39SF2A5, 40SM1A1, and 44SM2A7) prepared as described in Example 1 were analyzed for their HLA-DR restriction fashions by the examination of the reactivity to the RA antigen-presenting self or nonself RA synoviocytes, and to the antigen-presenting cell without the RA antigen. Antigen-presenting cells (PB-APC) from peripheral bloods of RA patients or healthy donors were used as the antigen-presenting cell without the RA antigen,

The T cell clones were examined in their proliferating activity by coculturing with the self or nonself RA synoviocytes or PB-APC from the RA patients or healthy donors (HD) in RPMI 1640 medium supplemented with 10% Fetal calf serum and antibiotics at 37°C in 5% CO₂ atmosphere. The results are shown in Table 1.

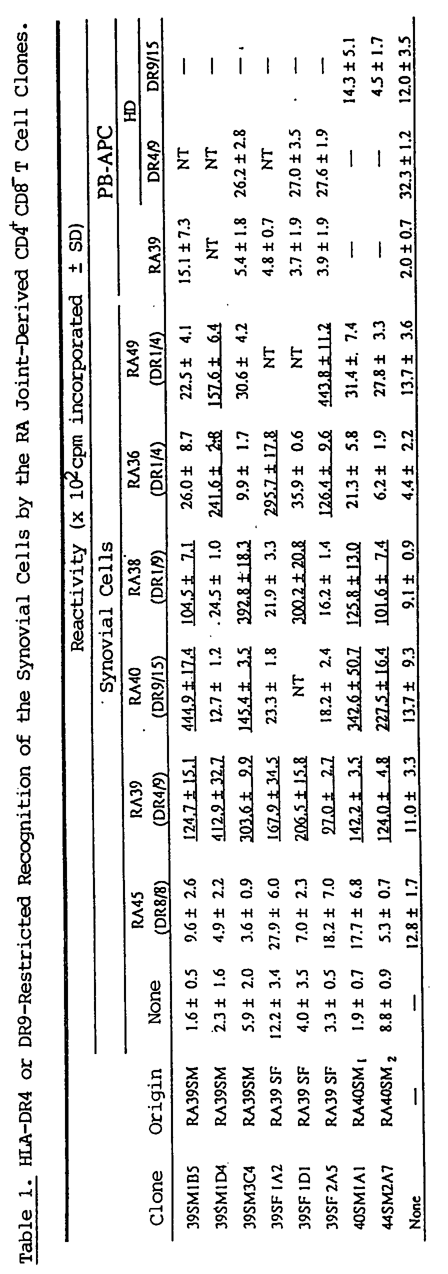

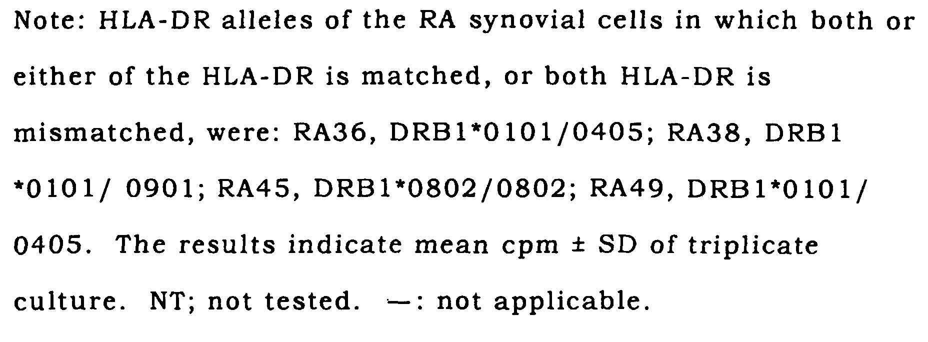

Table 1 indicates the following:
1) The clone from RA 39 proliferated in the presence of the autologous synovial cells bearing HLA-DR4 and -DR9, and were also reactive to the HLA-DR-matched nonself RA synovial cells;
2) 39SM1B5, 39SM3C4 and 39SM1D1 were reactive to the synovial cells bearing HLA-DR9, and 39SM1D4, 39SF1A2 and 39SF2A5 were reactive to the synovial cells bearing HLA-DR4, showing that the reactions are restricted with either of the HLA-DRs matched with the self;
3) The clone which proliferated in HLA-DR9-restricted manner was obtained from RA40SM₁ and RA 40SM₂ bearing HLA-DR9 and 15; and
4) The T cell clone reactive to the RA synovial cells were reactive to neither self PB-APC nor HLA-DR-matched PB-APC.

The above results show that the T cell clones of the present invention recognize the antigen existing exclusively on the RA synovial tissue in HLA-DR-restricted manner. Further, the fact that one clone of the invention can be reactive to various RA synovial cells may lead the presumption that possible antigen peptides presented on the HLA-DR of the RA synovial cells would be similar or identical each other. As such, the present inventors, for the first time, have obtained the data showing that the RA-associated antigen would be a single material.

### (2) Contribution of HLA-DR and TCR αβ molecule toward the recognition of RA synoviocyte by the T cell clone

The autoreactive T cell clones and the autologous synovial cells treated with anti-HLA-DR mAb (monoclonal antibody), or the autologous synovial cells and the T cell clones treated with anti-TCR αβ mAb (monoclonal antibody) were cocultured on the condition described in the above (1), the antibodies being remained in the media through the experiments. For control, the synovial cells were treated with irrelevant mouse IgG, and cocultured with the T cell clones, to determine the proliferating activity (autoreactivity) of the T cell clones. The results are shown in Figure 2. The results indicate mean cpm ± SD of triplicate culture. The symbol "#" shows not tested.

Figure 2 indicates the followings;
1) The anti-HLA-DR and the anti-TCR αβ antibodies blocked the autoreactivity of the T cell clones; and
2) The irrelevant mouse IgG did not affected the reactivity. Treatment of the T cell clones with the irrelevant mouse IgG did not alter the results (data not shown).

The above results show that the autoreactive T cell clones proliferate through recognizing specifically the antigen presented on HLA-DR of the synovial cells via the TCR, rather than through the stimulation with non-specific adhesion molecules.

### (3) Examination of TCR structure

The structure of the receptor involving the recognition of the RA synovial cells by the autoreactive T cell clones was examined. The nucleotide sequences of the V, D, and J regions in the TCR was determined according to the adaptor-ligation-mediated PCR (Y. Tsuruta, et al., J. Immunol. Methods 161, 7; 1993) and the chain termination method, and their amino acid sequences were determined on the basis of the nucleotide sequence. The V and J regions in the TCR are named by the nomenclature of Koop (B.F.Koop et al., Genomics 19, 478; 1994, N. Kimura et al., Eur. J. Immunol. 17, 375; 1987, B. Toyonaga et al., Proc. Natl. Acad. Sci. USA 82, 8624; 1985, S. Roman-Roman et al., Eur. J. Immunol. 21, 935; 1991, M. Yassai et al., Human Immunol. 34, 279; 1992). The results are shown in Table 2.

**Table 2**

| Comparison of Amino Acid Sequences of the TCR of the Autoreative T Cell Clones TCR α chains | | | | |
|---|---|---|---|---|
| Clone | V | N | J | |
| 39SF 1A2 Vα | 23.1 ...GDSATYL CAV | R | TGNQFY FGTGTSLTVIPN | Jα 49 |
| 40SM1A1 Vα | 30.1 ...TDVGTYF CAG | R | NAGGTSYGKLT FGQGTILTVHPN | Jα 52 |
| 44SM2A7 Vα | 7.1 ...KDSASYF CAV | R | NNNAGNMLT FGGGTRLMVKPH | Jα 39 |
| 39SF 1D1 Vα | 2.3 ...SDSATYL CAV | RS | GGFKTI FGAGTRLFVKAN | Jα 9 |
| 39SM3C4 Vα | 14.1...GDAAMYF CAY | RSH | SGGSNYKLT FGKGTLLTVNPN | Jα 53 |
| 39SM1B5 Vα | 14.2 ...GDTAMY CAF | MKHV | GGSQGNLI FGKGTKLSVKPN | Jα 42 |
| 39SM1D4 Vα | 11.1...ADAAVYY CAV | EV | SGGYNKLI FGAGTRLAVHPY | Jα 4 |
| 39SF 2A5 Vα | 26.1 ...SHAGIYL CGA | DMY | TGFQKLV FGTGTRLLVSPN | Jα 8 |

| TCR β chains | | | | |
|---|---|---|---|---|
| Clone | V | NDN | J | |
| 39SF 1A2 Vβ | 6.7 ...EDSAVYLCASS | LAGTRTP | YEQY FGPGTRLTVT | Jβ 2.7 |
| 40SM1A1 Vβ | 6.7 ...EDSAVYLCASS | LGGLAE | ETQY FGPGTRLLVL | Jβ 2.5 |
| 44SM2A7 Vβ | 6.7 ...EDSAVYLCASS | LVAIA | GELF FGEGSRLTVL | Jβ 2.2 |
| 39SF 1D1 Vβ | 6.5 ...GDSAMYLCASS | GDG | SYEQY FGPGTRLTVT | Jβ 2.7 |
| 39SM3C4 Vβ | 6.3 ...EDSAVYLCASS | QRDG | NTGELF FGEGTRLTVL | Jβ 2.2 |
| 39SM1B5 Vβ | 12.2 ...SQTSVYFCAIS | ESROGGG | QPQH FGDGTRLSIL | Jβ 1.5 |
| 39SM1D4 Vβ | 12.2 ...SQTSVYFCAIS | GTGTGD | GYT FGSGTRLTVV | Jβ 1.2 |
| 39SF 2A5 Vβ | 12.2 ...SQTSVYFCAIS | SGTGRGE | EKLF FGSGTQLSVL | Jβ 1.4 |
| Note: A, alanine; C, cysteine; D, asparatic acid; E, glutamic acid; F, phenylalanine; G, glycine; H, histidine; I, isoleucine; K, lysine; L, leucine; M, methionine; N, asparagine; P, proline; Q, glutamine; R, arginine; S, serine; T, threonine; V, valine; Y, thyrosin. The N region is formed by incorporation of nucleotide(s) between the V-D or the D-J (the β chain), or the V-J (the α chain) through a terminal deoxynucleotide transferase. | | | | |

Table 2 indicates the followings:
1) Among eight clones, five have arginine in their CDR3 regions (Complementarity Determining Region 3, antigen-binding portion containing the N region of the V-D-J or the V-J) of TCR Vα chains, and all of them use TCR Vβ6;
2) The remaining three clones have no significant characters in their α chains, whereas all of them use TCR Vβ12.

The above results show that the T cells having arginine in the a chain CDR3 regions, which provide TCR variability to respond to various antigens, are exclusively present in the RA synovial tissues, and that such T cells have the TCRβ chain containing a certain motif. Further, these also show that the RA-associated antigen peptide(s) have common characteristics in structure, and that the T cell clones of the present invention are useful in exploring the antigen's structure.

### (4) Reactivity of the autoreactive T cell clones to solubilized RA antigens

Solubilized antigens were prepared by treating the RA40 synoviocytes with hypertonic potassium chloride substantially according to the method of Meltzer (M.S.Meltzer et al., J. Nat. Cancer Inst. 47, 703; 1971). Actual procedures therefor are described as follow. Three ml of 3M KCL in 0.005M potassium phosphate buffer, pH7.4, was added to a cell pellet of 5 x 10⁷-1 x 10⁸ synovial cells. The antigen extraction was performed at 4°C for 16 hours, and the supernatant was dialyzed twice in deionized water at 4°C for a hour. The extract was filtered and stored at 4°C until use.

Then, the resultant solubilized antigens were cocultured with the autoreactive T cell clones in the presence or absence of PB-APC from healthy donors bearing HLA-DR9 (DRB1*0901) for 72 hours. Thereby, the proliferating activity of the T cell clones was determined on the basis of ³H-thymidine incorporation (CPM) to examine whether the autoreactive T cell clones were specifically reactive to the solubilized antigens presented on a certain HLA-DR. The results are shown in Table 3.

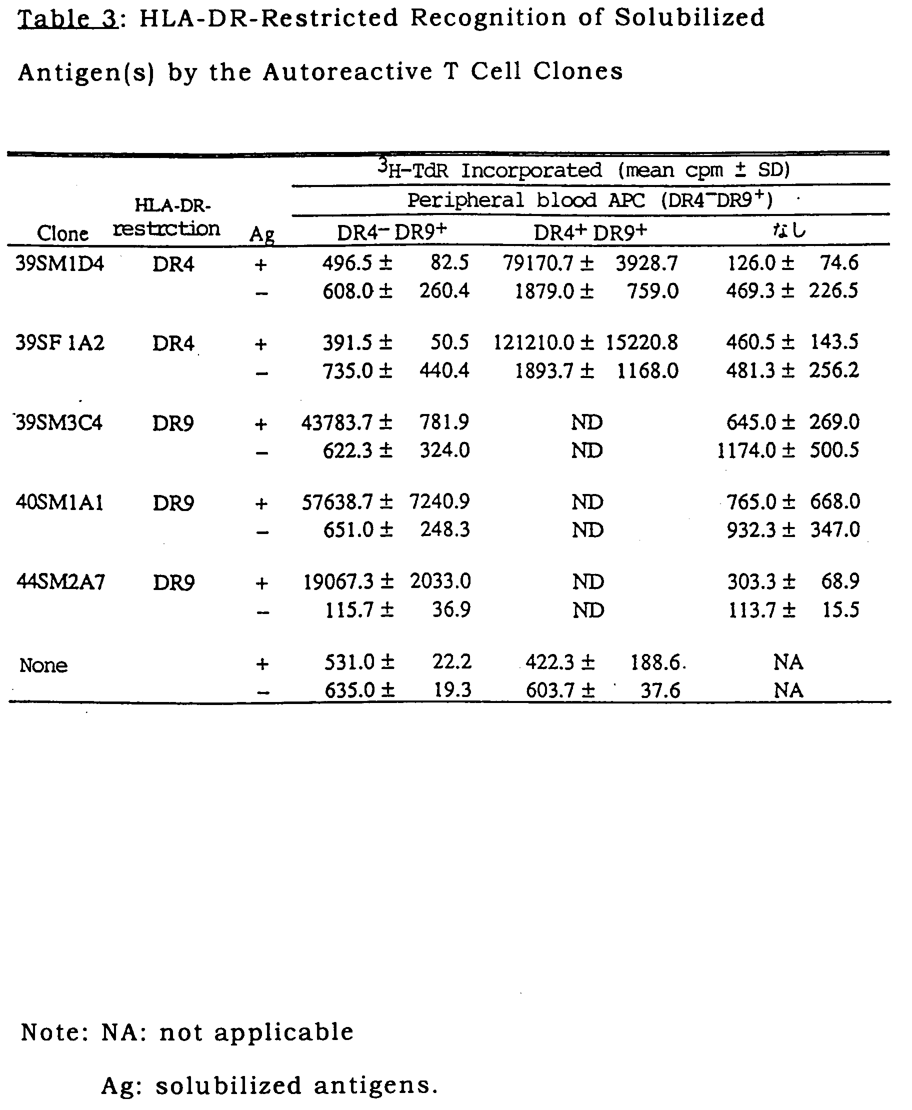

Table 3 shows the followings:
1) The clones restricted with HLA-DR9 of the RA synovial cells (39SM3C4, 40SM1A1, and 44SM2A7) proliferate in recognition of the solubilized antigens in the presence of HLA-DR9-positive PB-APC, and do not proliferate in the absence of HLA-DR9-positive PB-APC.
2) The clones restricted with HLA-DR4 of the RA synovial cells (39SM1D4 and 39SF1A2) do not recognize the solubilized antigens even in the presence of HLA-DR9-positive PB-APC. Similar examination was performed with the solubilized antigens extracted from the RA synovial cell and HLA-DR4-positive PB-APC, revealing that the HLA-DR4-restricted T cell clones proliferated in the presence of both antigens and PB-APC.

### (5) Specificity of the autoreactive T cell clones to RA synovial cells

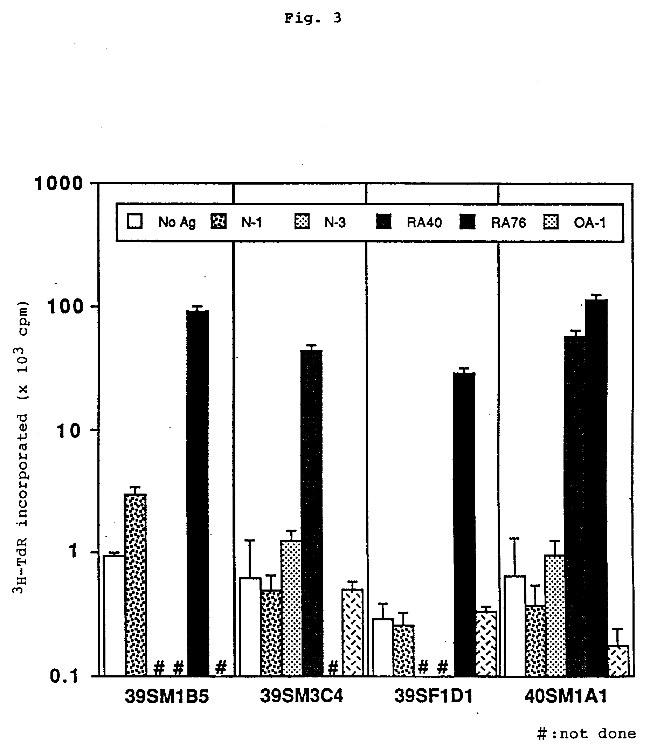

According to a similar method to that described in the above (9), solubilized antigens were extracted from synovial cells of healthy donors, and the proliferating activity of HLA-DR9-restricted autoreactive T cell clones (39SM1B5, 39SM3C4, 39SF1D1, 40SM1A1) was determined by culturing them in the presence or absence of HLA-DR9-positive PB-APC as shown above. The results are shown in Figure 3. Figure 3 shows that the T cell clones are reactive to the solubilized antigens from the RA synovial cells, whereas they are not reactive to the solubilized antigens from the synovial cells of non-RA patients (healthy donors (N-1 and N-2), and arthrosis deformans patients (OA-1)). This means that the antigens to be recognized by the autoreactive T cell clones of the present invention would exist exclusively in the RA synovial cells.

### (6) Identification of the solubilized RA antigens

The extract prepared in the above (4) was dialyzed in serum-free RPMI 1640 medium at 4°C for a hour, or was fractionated by gel-permeation HPLC. In the case of the latter, the solubilized antigens extracted from RA synovia (1 mg/ml) were fractionated by gel-permeation HPLC over TSK gel G3000SW column (7.5 x 600mm, 10µm, Tosoh Co., Tokyo) eluting with PBS at 0.5 ml/minute. Each fraction was filtered, and the reactivity of the autoreactive T cell clones (proliferating activity) to the chromatography fractions containing the solubilized antigens was examined in according to a similar method to that of Experiment 1 (1). Briefly, each fraction was cocultured with RA synovial cell's HLA-DR-restricted T cell clones (39SM1B5, 39SM1D4, 39SM3C4, 39SF1A2, 39SF2A5, or 40SM1A1) and PB-APC bearing the coincident DR in PRMI1640 medium supplemented with 10% Fetal calf serum and antibiotics at 37°C in a 5% CO₂ atmosphere, to examine the proliferating activity, and the fractions which were found to contain the proliferating activity were further examined to determine the molecular weight of the possible antigens therein. Typical results are shown in Figure 4. As shown in Figure 4b, HLA-DR9-restricted T cell clone, 39SM1B5 and HLA-DR4-restricted T cell clone, 39SF2A5 proliferate in recognition of HPLC fractions 31 and 38 containing the solubilized antigens, respectively. The molecular weights of the solubilized antigens therein were estimated to be 50 kDa and 25 kDa, respectively, by calibration curve determined from the elution points of the standards of BSA (bovine serum albumin), OVA (ovalbumin), CA (carbonic anhydrase), STI (soybean trypsin inhibitor), and ALA (bovine milk α-lactoglobulin) (Figure 4a).

It should be noted that the other HLA-DR-restricted clones, 39SM3C4, 39SF1A2, 39SF2A5 and 40SM1A1 also proliferate in response to these fractions (data not shown).

## Claims

1. A human T cell clone which recognizes a rheumatoid arthritis-associated antigen derived from synovial cells of patients suffering from rheumatoid arthritis in HLA-DR-restricted manner.

2. The human T cell clone as claimed in Claim 1 in which the clone is not reactive to the peripheral blood antigen-presenting cells derived from said patients suffering from rheumatoid arthritis when the cells are associated with nothing.

3. The human T cell clone as claimed in Claim 1 in which the clone is HLA-DR4- or HLA-DR9-restricted.

4. The human T cell clone as claimed in Claim 1 in which the CDR3 region in the T cell receptor α chain has arginine.

5. The human T cell clone as claimed in Claim 1 in which the T cell receptor β chain is Vβ 6 family.

6. The human T cell clone as claimed in Claim 1 in which the T cell receptor β chain is Vβ 12 family.

7. The human T cell clone as claimed in Claim 1 in which the clone is CD4-positive, and proliferates in the presence of the synovial cells from rheumatoid arthritis patient bearing HLA-DR4 or HLA-DR9.

8. A process for preparing the human T cell clone as claimed in any one of Claims 1 to 7, which comprises:
(1) establishing T cell clones derived from synovial tissues or synovial fluids of a rheumatoid arthritis patient;
(2) selecting the T cell clones reactive to the autologous synovial cells from the same patient;
(3) selecting the T cell clones which are not reactive to synovial cells derived from another rheumatoid arthritis patient which bears the different HLA-DR from the autologous synovial cells; and/or
(4) selecting the T cell clones which are not reactive to cells expressing the same HLA-DR as the autologous synovial cells, which cells do not express the rheumatoid arthritis-associated antigen derived from the autologous synovial cells.

9. A process for preparing an autoreactive T cell clone, which comprises adding cells which may be an antigen-presenting cell and solubilized material of the synovial cells to T cells which proliferate only in response to the antigen, culturing the mixture in an appropriate medium to establish T cell lines, and then performing each step as described in (3) and (4) of Claim 8.

10. A method for detecting a rheumatoid arthritis-associated antigen, in which the reactivity of the T cell clone as claimed in any one of Claims 1 to 6 is used as an indicator.

11. The method for detecting a rheumatoid arthritis-associated antigen, which comprises obtaining synovial cells from a patient suspected to have rheumatoid arthritis, solubilizing the synovial cells, and reacting the solubilized material with the T cell clone as claimed in any one of Claims 1 to 6 and the cells derived from the patient which may be an antigen-presenting cell so as to examine the reactivity of the clone.

12. A rheumatoid arthritis-associated antigen from synovial cells which are reactive to the human T cell clone as claimed in any one of Claims 1 to 6.

13. The rheumatoid arthritis-associated antigen as claimed in Claim 12, which is estimated as 25 kDa or 50 kDa of molecular weight by determination on HPLC over TSK gel G3000SW column eluting with PBS at 0.5 ml/minute.

14. A process for screening for an agonist or an antagonist of the rheumatoid arthritis-associated antigen as claimed in Claim 12, which comprises reacting said antigen with a sample suspected to contain said agonist or antagonist, and examining the reactivity of the clone.

15. A medium which comprises human interleukin-2, a culture supernatant of PHA-stimulated human peripheral blood mononuclear cell, and fatal calf serum.
